# EUROPEAN PATENT APPLICATION

(11) **EP 4 252 667 A1**
(43) Date of publication of application: **04.10.2023**
(21) Application number: 22167807.1
(22) Date of filing: 12.04.2022
(51) Int. Cl.: A61B 8/00

(54) **SYSTEM AND METHOD FOR GENERATING AN ULTRASOUND DEVICE INTERACTION INDICATOR**

(30) Priority: 31.03.2022 US 202263325654 P
(71) Applicant: Koninklijke Philips N.V., 5656 AG Eindhoven (NL)
(72) Inventor: SUTTON, Jonathan Thomas, Eindhoven (NL); POLAND, McKee Dunn, Eindhoven (NL); KYNE, Sean Joseph, Eindhoven (NL); ZHENG, Mingxin, Eindhoven (NL)
(74) Representative: Philips Intellectual Property & Standards

(57) **Abstract**

A system and method for generating an ultrasound device interaction indicator representative of a force or pressure exerted by an ultrasound device and/or an orientation of the ultrasound device relative to the subject. An acoustically translucent standoff layer is positioned between the ultrasound device and the subject. Ultrasound echoes received by the ultrasound device, including ultrasound echoes from the standoff layer, are processed to determine a deformation of the standoff layer caused by an axial force or pressure exerted by the ultrasound device. The deformation of the standoff layer is used, along with characteristics of the standoff layer, to generate the ultrasound device interaction indicator.

## Description

### FIELD OF THE INVENTION

The invention relates to the field of ultrasound imaging, and, in particular, to point-of-care ultrasound imaging.

### BACKGROUND OF THE INVENTION

Point-of-care ultrasound imaging is becoming increasingly popular as technological advances, such as smaller form factors, low cost systems, artificial intelligence, and the digital cloud, improve the practicality and reduce the cost of point-of-care ultrasound.

The increase in point-of-care ultrasound has led to a number of new ultrasound applications, such as patches for longitudinal monitoring, and hemodynamic monitoring (e.g. non-invasive blood pressure estimation).

One issue with point-of-care ultrasound is that it is generally carried out by clinicians with little to no training in ultrasound imaging. Such clinicians are less skilled at reducing disturbance to a subject during the imaging procedure, and may not be able to judge when an appropriate amount of force is being applied when coupling an ultrasound probe to the skin. Insufficient force can lead to suboptimal images and/or image artifacts. On the other hand, an extended period of contact between an ultrasound probe or patch and a subject's body can cause pressure ulcers. This is particularly a problem for patch-based applications, which are often designed for monitoring a subject over time.

There is therefore a need for improved information relating to the force and/or pressure of an ultrasound transducer on a subject.

### SUMMARY OF THE INVENTION

The invention is defined by the claims.

According to examples in accordance with an aspect of the invention, there is provided a processing system for generating an ultrasound device interaction indicator representative of a force or pressure exerted by an ultrasound device on a subject and/or an orientation of the ultrasound device relative to the subject.

The processing system is configured to: obtain, at an input interface, one or more characteristics of an acoustically translucent standoff layer between a surface of the ultrasound device and the subject's skin; obtain, at the input interface, an ultrasound signal from the ultrasound device, wherein the ultrasound signal is representative of a plurality of ultrasound echoes received by the ultrasound device from different depths for each of one or more scan lines; process the ultrasound signal to determine a measure of deformation of the acoustically translucent standoff layer; and process the measure of deformation and the one or more characteristics of the acoustically translucent standoff layer to generate the ultrasound device interaction indicator.

The inventors have recognized that the use of an acoustically translucent standoff layer allows an indication of the force/pressure exerted by an ultrasound device on a subject when the ultrasound device is coupled to the subject to be generated, by determining a measure of the deformation of the acoustically translucent standoff layer caused by the force exerted by the ultrasound device.

The inventors have further recognized that a measure of deformation or an indication of force or pressure across the ultrasound device may be used to provide information about the orientation of the ultrasound device, by identifying a difference in the measure of deformation or force/pressure across the acoustically translucent standoff layer (i.e. in a direction perpendicular to an imaging direction). A tilted position of an ultrasound device (i.e. with the imaging axis not perpendicular to the subject's skin) results in reduced image quality, as the effective aperture width is truncated and the beamformed signal summation is diminished. It is therefore desirable to provide information that would allow a user to determine whether the orientation of the ultrasound device is correct.

This method for generating an ultrasound device interaction indicator uses ultrasound echoes received as part of the operation of the ultrasound device, avoiding the need for a separate sensor to measure the force or pressure exerted by the ultrasound device.

An acoustically translucent standoff layer is a layer positioned between the ultrasound imaging device and the subject's skin that has sufficient acoustic transparency so as not to significantly reduce a quality of images produced by the device.

The ultrasound device may, for example, be a hand-held ultrasound probe or an ultrasound patch.

In some examples, the generated ultrasound device interaction indicator is output at an output interface. The ultrasound device interaction indicator may, for instance, be used to control or define a user-perceptible output that is provided to an output user interface.

In some examples, the interaction indicator is not on the display for the clinician but consumed by the ultrasound system internally. Based on the applied force, the ultrasound system may change behavior such as changing transmit beam angles adaptively, changing system power settings or TGC gain settings, activating imaging modes, alerting the users that pressure is not suitable for an elastography scan, etc.

In some examples, the one or more characteristics of the acoustically translucent standoff layer comprise at least one of: a thickness in a zero force state a thickness at a predefined non-zero compression force or pressure, and/or an elastic modulus.

The one or more characteristics of the acoustically translucent standoff layer may further comprise a hysteresis behavior.

In some examples, the processing system is configured to determine the measure of deformation by: identifying a plurality of ultrasound echoes from each of the one or more scan lines in the ultrasound signal; processing, for each scan line, the plurality of echoes from the scan line to determine a depth of a distal surface of the acoustically translucent standoff layer, wherein the distal surface of the acoustically translucent standoff layer is distal to the ultrasound device; and processing the depth of the distal surface for each of the one or more scan lines to determine the measure of deformation.

This method is based on the recognition that deformation of the acoustically translucent standoff layer will affect the thickness of the acoustically translucent standoff layer (and therefore the depth at which the distal surface is detected).

The distal surface of the acoustically translucent standoff layer is a surface furthest from the ultrasound device (i.e. a skin-facing surface of the acoustically translucent standoff layer). This surface is at a boundary with an adjacent layer, which may be the subject's skin or a layer disposed between the acoustically translucent standoff layer and the subject's skin (e.g. an adhesive, a sterile film, air, water, sweat, etc.), and is detectable due to the difference in acoustic impedance between the acoustically translucent standoff layer and the adjacent layer.

The processing system may be configured to determine the depth of the distal surface for each scan line by: processing the plurality of echoes from the scan line to identify an echo for which a signal intensity value first exceeds an intensity threshold; and selecting a depth of the identified echo as the depth of the distal surface.

The intensity threshold may be a predetermined threshold, or a threshold determined by the processing system based on the ultrasound signal (e.g. based on signal intensity values of the ultrasound signal).

For example, the intensity threshold may have a predetermined value that has been determined based on characteristics of the ultrasound device and the acoustically translucent standoff layer (e.g. the acoustic transparency of the acoustically translucent standoff layer).

In some examples, the processing system is configured to determine the measure of deformation by: processing the ultrasound signal to generate an image comprising at least part of the acoustically translucent standoff layer; and performing a cross-correlation of the generated image with a reference image to determine the measure of deformation.

The reference image may be an image of the acoustically translucent standoff layer when no force is applied. The cross-correlation may be a series of 1D A-line correlations or a 2D cross-correlation.

In some examples, the generated image may be processed by performing, for example, axial interpolation and/or azimuthal decimation before performing the cross-correlation. The image processing parameters may be defined based on an imaging frequency of the ultrasound device and one or more characteristics of the acoustically translucent standoff layer.

The processing system is configured to generate the ultrasound device interaction indicator by selecting one of a plurality of predetermined ultrasound device interaction indicators.

In other words, the ultrasound device interaction indicator may be a binary or categorical indicator. This may be particularly beneficial in point-of-care ultrasound applications, where a user may lack the expertise to interpret a numerical value of force/pressure/orientation angle.

The predetermined ultrasound device interaction indicators may have varying levels of granularity. For example, the ultrasound device interaction indicators may represent three states: a first state in which a force or pressure is below a first threshold, a second state in which the force or pressure exceeds the first threshold but is below a second, higher threshold, and a third state in which the force or pressure exceeds the second threshold.

The indication may, for example, be a textual indication (e.g. "pressure too low", "pressure good" and "pressure too high"). In another example, different colors may be used to represent different states.

The ultrasound device interaction indicator may be a numerical value of the force or pressure exerted by the ultrasound device on the subject.

Where the ultrasound interaction device indicator is provided to an output user interface, a numerical value allows a clinician to make their own assessment as to whether the force/pressure exerted by the ultrasound device or the orientation angle is appropriate, and also enables them to determine the change required if the force/pressure is either insufficient or excessive or if the orientation angle is inappropriate.

In some examples, the clinician may choose a level of granularity of the ultrasound device interaction indicator (e.g. the clinician may choose between a categorical indication and an indication comprising a numerical value of force/pressure/orientation angle). In other examples, the level of granularity may be selected automatically according to the type of ultrasound application.

In some examples, the ultrasound device interaction indicator may comprise both a predetermined ultrasound device interaction indicator and a numerical value.

A numerical value of force, pressure or orientation angle may also be used internally by the processing system, e.g. to control the ultrasound system.

In some examples, the processing system is further configured to: process the one or more characteristics of the acoustically translucent standoff layer and the ultrasound signal to determine a measure of strain hysteresis of the acoustically translucent standoff layer; and, in response to a determination that the measure of strain hysteresis exceeds a predetermined hysteresis threshold, generate an indication that the acoustically translucent standoff layer needs replacing, and output, at an output interface, the indication that the acoustically translucent standoff layer needs replacing.

The strain hysteresis of hydrogels increases with successive force applications. Identifying when the hysteresis effect is great enough to affect the results of deformation determination allows users of the processing system to replace the acoustically translucent standoff layer when this occurs.

There is also proposed a system for providing an ultrasound device interaction indicator representative of a force or pressure exerted by an ultrasound device on a subject. The system comprises: an ultrasound device configured to transmit and receive ultrasound, and to generate the ultrasound signal representative of a plurality of received ultrasound from different depths for each of one or more scan lines; an acoustically translucent standoff layer positioned between a surface of the ultrasound device and the subject's skin; and the processing system described above.

In some examples, the acoustically translucent standoff layer comprises an aqueous gel pad.

Aqueous gel pads are known in the art and widely available, so may easily be procured according to user specifications.

In some examples, the acoustically translucent standoff layer comprises coupling means for fastening the acoustically translucent standoff layer to the ultrasound device.

This enables quick installation of the acoustically translucent standoff layer on the ultrasound device, and may aid a clinician in positioning the acoustically translucent standoff layer appropriately.

The acoustically translucent standoff layer may, for example, include a mechanical fastener, such as a clip, or an adhesive "skirt" surrounding the perimeter of the acoustically translucent standoff layer.

A thickness of the acoustically translucent standoff layer may be selected according to a target imaging anatomy.

A desirable thickness of the acoustically translucent standoff layer depends on the imaging application.

According to another aspect of the invention, there is provided a computer-implemented method for generating an ultrasound device interaction indicator representative of a force or pressure exerted by an ultrasound device on a subject and/or an orientation of the ultrasound device relative to the subject.

The computer-implemented method comprises: obtaining, at an input interface, one or more characteristics of an acoustically translucent standoff layer between a surface of the ultrasound device and the subject's skin; obtaining, at the input interface, an ultrasound signal from the ultrasound device, wherein the ultrasound signal is representative of a plurality of ultrasound echoes received by the ultrasound device from different depths for each of one or more scan lines; processing the ultrasound signal to determine a measure of deformation of the acoustically translucent standoff layer; and processing the measure of deformation and the one or more characteristics of the acoustically translucent standoff layer to generate an ultrasound device interaction indicator.

There is also proposed a computer program product comprising computer program code means which, when executed on a computer device having a processing system, cause the processing system to perform all of the steps of the method described above.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an exemplary ultrasound system;
Fig. 2 illustrates a system for providing an ultrasound device interaction indicator, according to an embodiment of the invention;
Fig. 3A illustrates a method for determining a measure of deformation of an acoustically translucent standoff layer, according to an embodiment of the invention;
Fig. 3B illustrates how the method of Figure 3A may be used to determine a measure of deformation;
Fig. 4 illustrates a method for determining a measure of deformation of an acoustically translucent standoff layer, according to another embodiment of the invention;
Fig. 5 illustrates example outputs corresponding to ultrasound device interaction indicators;
Fig. 6 illustrates an example acoustically translucent standoff layer comprising coupling means; and
Fig. 7 illustrates a computer-implemented method for generating an ultrasound device interaction indicator, according to an embodiment of the invention.

### DETAILED DESCRIPTION OF THE EMBODIMENTS

The invention will now be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for generating an ultrasound device interaction indicator representative of a force or pressure exerted by an ultrasound device and/or an orientation of the ultrasound device relative to the subject. An acoustically translucent standoff layer is positioned between the ultrasound device and the subject. Ultrasound echoes received by the ultrasound device, including ultrasound echoes from the standoff layer, are processed to determine a deformation of the standoff layer caused by an axial force or pressure exerted by the ultrasound device. The deformation of the standoff layer is used, along with characteristics of the standoff layer, to generate the ultrasound device interaction indicator.

Embodiments are at least partly based on the realization that a force/pressure exerted by an ultrasound device on a subject would deform a standoff layer between the ultrasound device and the subject, and that ultrasound imaging data obtained by the device may be used to determine the deformation of the standoff, and therefore the force/pressure exerted by the ultrasound device. In particular, the inventors have recognized that measuring the deformation of a standoff layer with known properties provides a more reliable indicator of force or pressure than measuring tissue (e.g. skin) deformation, as the properties of tissue vary from subject to subject and further depend on environmental conditions etc. The inventors have further recognized that the deformation or force/pressure across the standoff layer provides information about the orientation of the ultrasound device.

Illustrative embodiments may, for example, be employed in ultrasound imaging systems, such as point-of-care ultrasound systems.

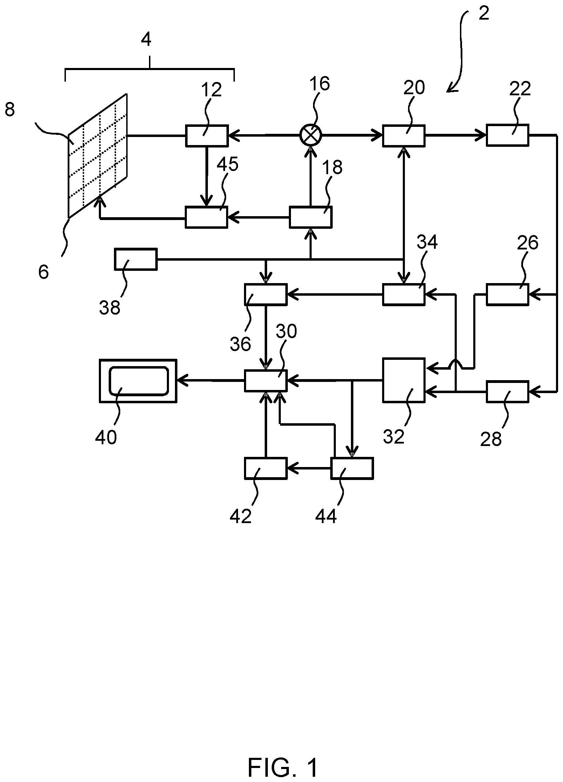

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

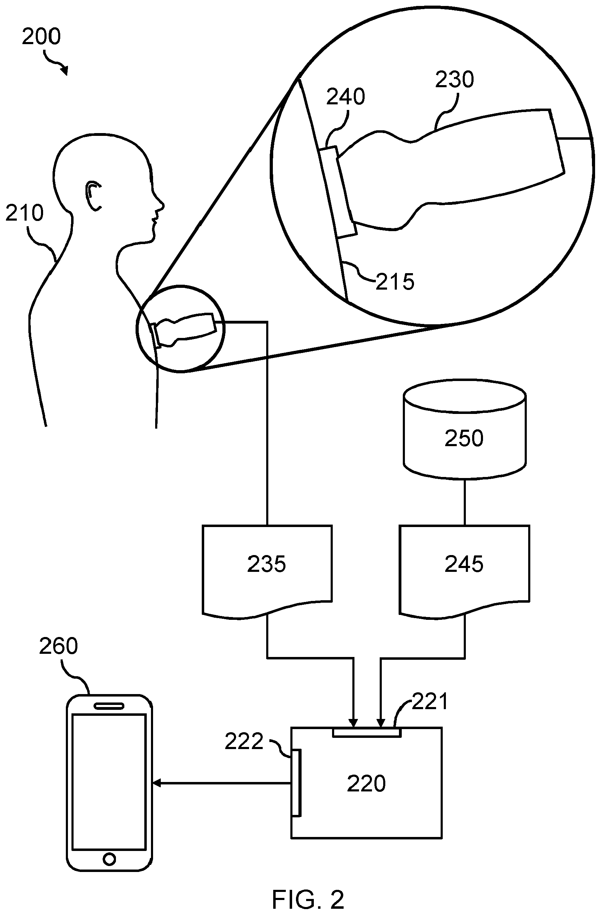

Fig. 2 illustrates a system 200 for generating an ultrasound device interaction indicator, according to an embodiment of the invention. An ultrasound device interaction indicator is an indicator representative of a force or pressure exerted by an ultrasound device on a subject 210, and/or an orientation of the ultrasound device relative to the subject. The system 200 comprises a processing system 220, an ultrasound device 230 and an acoustically translucent standoff layer 240. The processing system is, itself, an embodiment of the invention.

The acoustically translucent standoff layer 240 is positioned between a surface of the ultrasound device 230 and the subject's skin 215. The acoustically translucent standoff layer is a layer of material that has an acoustic transparency that allows ultrasound imaging through the material (i.e. imaging of structures beyond the material). Suitable materials for the acoustically translucent standoff layer will be readily apparent to the skilled person. For example, the acoustically translucent standoff layer may comprise an aqueous gel pad (e.g. an aqueous hydrogel pad). Aqueous gel pads are known in the art and are commonly used to provide acoustic coupling between an ultrasound device and skin when imaging difficult-to-visualize areas. The acoustically translucent standoff layer is described in more detail below.

The processing system 220 obtains, at or from an input interface 221, one or more characteristics 245 of the acoustically translucent standoff layer 240. The one or more characteristics of the acoustically translucent standoff layer are characteristics that may be used, in combination with a measure of deformation, to generate an ultrasound device interaction indicator representative of a force, pressure and/or orientation. For example, the one or more characteristics of the acoustically translucent standoff layer may comprise at least one of: a thickness in a zero force state, a thickness at a predefined non-zero compression force or pressure, and/or an elastic modulus. The use of these characteristics to generate the ultrasound device interaction indicator is described in more detail below.

The one or more characteristics 245 of the acoustically translucent standoff layer 240 may be obtained from a memory unit 250, as shown in Fig. 2. In some examples, some or all of the one or more characteristics may be obtained via user input from a user input device.

The ultrasound device 230 is a device capable of transmitting and receiving ultrasound. Any suitable ultrasound device may be used, including handheld ultrasound probes and ultrasound patches. The system 200 is particularly beneficial for point-of-care ultrasound applications, which are often carried out by users with less skill in determining an appropriate amount of pressure to apply themselves.

The ultrasound device 230 generates an ultrasound signal 235 representative of a plurality of ultrasound echoes received by the ultrasound device from different depths for each of one or more scan lines, and the processing system 220 obtains the ultrasound signal from the ultrasound device at or from the input interface 221.

The different depths may include at least one depth corresponding to the acoustically translucent standoff layer and at least one depth corresponding to a layer beyond the acoustically translucent standoff layer (i.e. a layer on the opposite side of the acoustically translucent standoff layer to the ultrasound device, e.g. a skin layer). In other words, the received ultrasound may include echoes from the acoustically translucent standoff layer and echoes from another layer that have been transmitted through the acoustically translucent standoff layer.

The processing system 220 then processes the ultrasound signal 235 to determine a measure of deformation of the acoustically translucent standoff layer 240. A measure of deformation may, for example, comprise an axial strain and/or a deformed thickness of the acoustically translucent standoff layer.

In some examples, the processing system determines the measure of deformation based on a depth of a distal surface of the acoustically translucent standoff layer 240 (i.e. a surface furthest from the ultrasound device 230). In other words, the measure of deformation may be determined based on a depth of a boundary between the acoustically translucent standoff layer and a layer on the opposite side of the acoustically translucent standoff layer to the ultrasound device (e.g. the subject's skin 215). In other examples, the processing system determines the measure of deformation based on cross-correlation with a reference image.

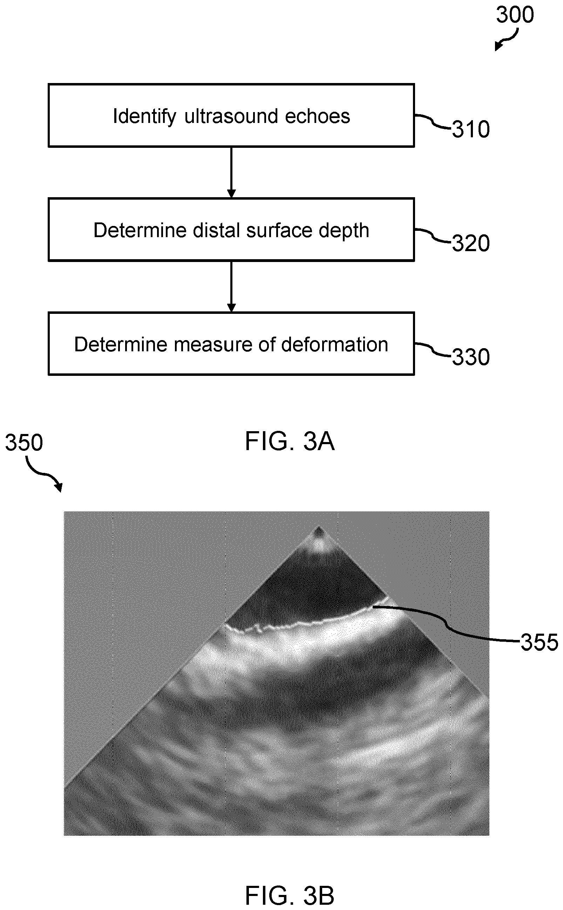

Fig. 3A illustrates a method 300 for determining a measure of deformation of an acoustically translucent standoff layer, according to an embodiment of the invention. In method 300, the processing system 220 determines the measure of deformation based on a depth of a distal surface of the acoustically translucent standoff layer (i.e. a skin-facing surface). This method provides a simple way to determine a measure of deformation at a data detection stage, which may easily be implemented in, for example, a field-programmable gate array.

The method 300 begins at step 310, in which a plurality of ultrasound echoes are identified in the ultrasound signal 235 for each of the one or more scan lines. Methods of identifying ultrasound echoes in an ultrasound signal will be apparent to the skilled person.

At step 320, the plurality of echoes from each scan line are processed to determine, for each scan line, a depth of a distal surface of the acoustically translucent standoff layer 240.

The depth of the distal surface for a particular scan line may be determined by processing the plurality of echoes from the scan line to identify an echo for which a signal intensity value first exceeds an intensity threshold. Since an adjacent layer to the distal surface (e.g. skin, adhesive, air, etc.) will generally have a different acoustic impedance to the acoustically translucent standoff layer, the boundary between the acoustically translucent standoff layer and an adjacent layer will be characterized by an upward transition in signal intensity.

The plurality of echoes may be processed in order of increasing depth; the first echo to be identified as having a signal intensity value exceeding the predetermined intensity threshold is then selected as the echo corresponding to the distal surface for the scan line. The depth of the identified echo is selected as the depth of the distal surface for the scan line. This process is repeated for each scan line.

The intensity threshold may be a predetermined threshold, or a threshold determined based on the ultrasound signal 235. A predetermined intensity threshold may, for example, be determined based on the characteristics of both the ultrasound device 230 and the acoustically translucent standoff layer 240. In some examples, a plurality of predetermined intensity thresholds, each for a different combination of ultrasound device and acoustically translucent standoff layer may be stored in the memory unit 250, and a suitable predetermined intensity threshold may be selected based on user input from a user input device.

In some examples, the processing system 220 may determine a value for the intensity threshold by processing the ultrasound signal 235. This allows the distal surface depths to be identified without requiring additional information (such as characteristics of the acoustically translucent standoff layer and ultrasound device), and is not affected by user-applied overall gain changes.

The processing system may determine the intensity threshold based on signal intensity values of the ultrasound signal (e.g. based on an average minimum signal intensity and an average maximum signal intensity). For instance, the intensity threshold may be set at a value between the average minimum signal intensity and the average maximum signal intensity (e.g. at a midpoint between these signal intensities, or a value between the midpoint and the average maximum signal intensity).

The ultrasound signal 235 may be preprocessed before step 320. For example, automatic time gain compensation (TGC) may be used to auto-normalize the brightness across a plurality of scan lines at a set of depths, or through the depth of each scan line.

At step 330, the depth of the distal surface for each of the one of more scan lines is processed to determine the measure of deformation. The depth of the distal surface corresponds to a current thickness of the acoustically translucent standoff layer 240. In some examples, such as where the measure of deformation is or comprises an axial strain, the determination of the measure of deformation may be further based on the one or more characteristics 245 of the acoustically translucent standoff layer (e.g. a thickness in a zero force state). In other words, the distal surface depths may be used in conjunction with known properties of the acoustically translucent standoff layer to determine the deformation of the acoustically translucent standoff layer.

In some examples, where a large number of scan lines are used, the determined distal surface depths may be preprocessed before determining the measure of deformation. For instance, an outlier analysis may be applied to the determined distal surface depths in order to identify false positives, which may be excluded from the distal surface depths used to determine the measure of deformation.

The depth of the distal surface may be averaged across multiple scan lines before determining the measure of deformation; the number of scan lines over which the depth of the distal surface is averaged may depend on a desired level of granularity. In some examples, the number of scan lines over which the depth of the distal surface is averaged may be selected to produce one average depth per scan frame (e.g. the distal surface depth may be averaged across approximately 128 scan lines).

Fig. 3B shows a portion of an ultrasound image 350 illustrating the idea behind this approach. The layer closest to the ultrasound device is the acoustically translucent standoff layer; since this layer is largely anechoic, and the next layer is not, a sharp difference in signal intensity is observed at the boundary between these layers.

Line 355 delineates the boundary detected using greyscale thresholding (with a threshold at 75% of the difference between the average minimum signal intensity and average maximum signal intensity). The displacement of this boundary from the ultrasound-emitting surface of the ultrasound device gives the current thickness of the acoustically translucent standoff layer, which can then be used to determine the force or pressure exerted by the ultrasound device. In Fig. 3B, the displacement is greater at one side and decreases across the field-of-view. This is indicative of a tilted ultrasound device (i.e. an ultrasound device having an imaging axis that is not perpendicular to the surface of the subject's skin).

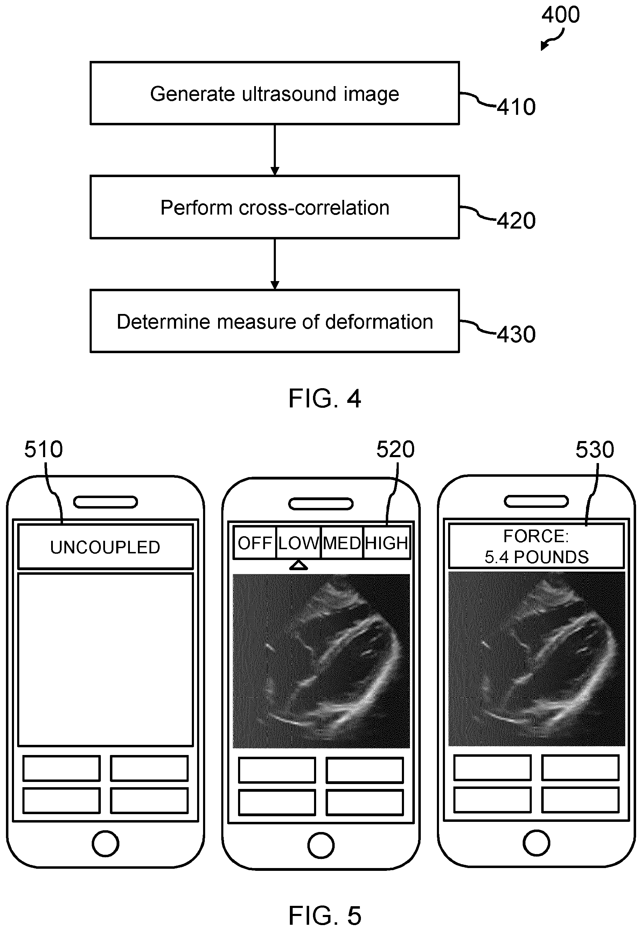

Fig. 4 illustrates another method 400 for determining a measure of deformation of an acoustically translucent standoff layer, according to an embodiment of the invention. In method 400, the processing system 220 determines the measure of deformation based on cross-correlation. This method may provide a more robust determination of the measure of deformation, and may, in particular, be used where the layer adjacent to the acoustically translucent standoff layer has a similar acoustic impedance to the acoustically translucent standoff layer (as in this case it is difficult to detect the distal surface of the standoff layer).

The method 400 begins at step 410, in which an ultrasound image is generated by processing the ultrasound signal 235. The ultrasound image is an image comprising at least part of the acoustically translucent standoff layer.

At step 420, a cross-correlation of the generated image with a reference image is performed. The reference image is an image that corresponds to an ultrasound image of the acoustically translucent standoff layer 240 when no force is deforming the acoustically translucent standoff layer (e.g. an image taken in air or otherwise obtained such that the pressure on the standoff layer is zero).

Cross-correlation is a well-known technique in the field of image processing, and can be used to measure a displacement of a structure between two images. See, for example, Bilgen and Insana (1996), "Deformation models and correlation analysis in elastography", J. Accoust. Soc. Am., 99(5):3212-3224; and Pinton et al. (2006), "Rapid tracking of small displacements using ultrasound", IEEE Transactions on Ultrasonics, Ferroelectrics, and Frequency Control, 53(6): 1103-1117.

By cross-correlating an ultrasound image of the deformed acoustically translucent standoff layer 240 with an ultrasound image of the non-deformed acoustically translucent standoff layer (i.e. when no force is applied), the displacement in the direction of the imaging axis (i.e. the axial strain) may be determined. The cross-correlation may be a series of 1D azimuthal line correlations or a 2D cross-correlation.

In some examples, the reference image may be obtained by a user of the ultrasound system imaging the acoustically translucent standoff layer 240. The user may be prompted to take this image, e.g. by outputting a user instruction to a user interface.

In other examples, the reference image may be generated from the ultrasound signal 235. The processing system may process the ultrasound signal to generate a plurality of frames, and the first frame having image contents (i.e. the first frame that is not entirely black) may be selected as the reference image, while a later frame may be selected as the ultrasound image that is cross-correlated with the reference image. This approach may be used where the ultrasound device 230 starts acquiring the ultrasound signal before the ultrasound device is pressed against the subject 210.

In yet other examples, the reference image may be a simulated image, generated from data representative of the acoustically translucent standoff layer when no force is applied.

The generated image (and/or the reference image) may be preprocessed before the cross-correlation is performed. Parameters used in the preprocessing may be selected based on the imaging frequency used by the ultrasound device 230 when acquiring the ultrasound signal 235 and one or more characteristics of the acoustically translucent standoff layer. Suitable image processing techniques for preprocessing the generated image, such as axial interpolation and azimuthal decimation, will be apparent to the skilled person.

At step 430, the measure of deformation is determined based on the results of the cross-correlation. The measure of deformation may be the axial strain, determined from the displacement in the direction of the imaging axis based on the cross-correlation.

Returning to Fig. 2, having determined a measure of deformation of the acoustically translucent standoff layer 240, the processing system 220 processes the measure of deformation and the one or more characteristics 245 of the acoustically translucent standoff layer to generate an ultrasound device interaction indicator. The ultrasound device interaction indicator is representative of a force or pressure exerted by the ultrasound device 230 on the subject 210 and/or an orientation of the ultrasound device relative to the subject.

The relationships between force/pressure and axial strain (or a deformed thickness) are very well known, so methods for generating an ultrasound device interaction indicator representative of a force or pressure exerted by the ultrasound device 230 based on a measure of deformation and one or more characteristics of the acoustically translucent standoff layer 240 will be readily apparent to the skilled person. Where the ultrasound device interaction indicator comprises a numerical value for pressure, the ultrasound device interaction indicator may be generated further based on a surface area of the ultrasound emitting surface of the ultrasound device (i.e. the surface in contact with or closest to the acoustically translucent standoff layer). The surface area may, for example, be obtained via user input or stored in the memory unit 250.

An ultrasound device interaction indicator representative of an orientation of the ultrasound device 230 relative to the subject 210 may be generated based on a difference in deformed thickness, force and/or pressure across the acoustically translucent standoff layer 240. A gradient in pressure, force or thickness also indicates the direction of the orientation of the probe. If the pressure, force or thickness is the same, or substantially the same, across the acoustically translucent standoff layer, this indicates that the imaging axis of the ultrasound device is perpendicular, or substantially perpendicular, to the subject's skin. If the deformed thickness is thinner, or the force/pressure is greater, at one side of the acoustically translucent standoff layer, this indicates that the ultrasound device is tilted towards this side of the acoustically translucent standoff layer.

The ultrasound device interaction indicator may be a numerical indicator (e.g. a numerical value of the force or pressure exerted by the ultrasound device 230 or of an angle of orientation of the ultrasound device), a categorical indicator (e.g. "pressure too high", "pressure correct", "pressure too low", "force too high", "force correct", "force too low", "orientation correct", "tilted left", "tilted right"), or a binary indicator (e.g. "coupled" or "uncoupled").

A categorical or binary ultrasound device interaction indicator may, for example, be generated by selecting one of a plurality of predetermined ultrasound device interaction indicators. One or more threshold values (i.e. a threshold for a pressure value, a force value or the measure of deformation) may be used to determine which of a plurality of predetermined ultrasound device interaction indicators to select.

For instance, where there are three predetermined ultrasound device interaction indicators (e.g. "pressure too low", "pressure correct" and "pressure too high"), the processing system 220 may select a first predetermined ultrasound indicator (e.g. "pressure too low") in response to a determination that a force, pressure or measure of deformation does not exceed a first predetermined threshold, a second predetermined ultrasound indicator (e.g. "pressure correct") in response to a determination that the force, pressure or measure of deformation exceeds the first predetermined threshold but does not exceed a second, higher predetermined threshold, and a third predetermine ultrasound device interaction indicator (e.g. "pressure too high") in response to a determination that the force, pressure or measure of deformation exceeds the second predetermined threshold.

In another example, where the ultrasound device interaction indicator is a binary indicator (e.g. "coupled" or "uncoupled"), a single threshold may be used: the processing system 220 may select a first predetermined ultrasound device interaction indicator (e.g. "uncoupled") in response to a determination that a force, pressure or measure of deformation does not exceed this threshold, and a second predetermined ultrasound device interaction indicator (e.g. "coupled") in response to a determination that the force, pressure or measure of deformation exceeds this threshold.

In some examples, the processing system 220 may determine one or more threshold values based on the one or more characteristics of the acoustically translucent standoff layer 240. For instance, where the one or more characteristics include at least one thickness at a predefined non-zero compression force or pressure, the predefined force or pressure may have been chosen as a threshold force or pressure. The thickness at the predefined force or pressure may then be defined as a threshold thickness, and measure of deformation comprising a deformed thickness of the acoustically translucent standoff layer may be compared with the threshold thickness. A categorical ultrasound device interaction indicator representative of force or pressure (e.g. of whether a force/pressure is too high, too low, or at a desired level) may thus be generated directly from a measurement of a (current) deformed thickness of the acoustically translucent standoff layer.

In some examples, the ultrasound device interaction indicator may be used to control or define a user-perceptible output, such as an audio and/or a visual indicator. For instance, the ultrasound device interaction indicator may be used to control or define a sound, a light, an image and/or a textual display.

The processing system 220 may output the ultrasound device interaction indicator, at an output interface 222, to an output user interface 260. In Fig. 2, the ultrasound device interaction indicator is provided to a smartphone display; however, the output user interface may be any device suitable for providing a user-perceptible output. For example, the output user interface may be or comprise a display device, a speaker or a light. In some examples, the output user interface may also be configured to receive user inputs (e.g. the output user interface may be a touchscreen device).

Fig. 5 illustrates example outputs corresponding to ultrasound device interaction indicators. The examples of Fig. 5 are output at a display of a smartphone or tablet as part of an ultrasound application, such as Philips Lumify.

Example output 510 comprises a textual display reading "Uncoupled". This output may be provided in response to the ultrasound device interaction indicator indicating that the ultrasound device 230 is exerting no force or pressure on the subject 210.

Example output 520 comprises a textual display including a plurality of possible force/pressure states ("Off', "Low", "Med" and "High") and an arrow indicating which state corresponds to the ultrasound device interaction indicator.

Example output 530 comprises a textual display providing a numerical value of the force exerted by the ultrasound device interaction indicator.

Further examples of suitable user-perceptible outputs controlled or defined by the ultrasound device interaction indicator will be apparent to the skilled person. For instance, color highlighting may be used to indicate which of a plurality of states corresponds to the ultrasound device interaction indicator, or a diagram may illustrate an orientation of the ultrasound device. In some examples, the ultrasound device interaction indicator may, in addition to being provided to an output user interface or alternatively, be used to control an operation of the ultrasound device 230. For instance, the processing system 220 may obtain measurements of an anatomical structure by controlling the ultrasound device to acquire ultrasound imaging data of the anatomical structure in response to a determination that a force or pressure exceeds a desired force or pressure. In another example, a determined force or pressure may be used as a threshold trigger in strain elastography.

As described above, the invention makes use of an acoustically translucent standoff layer to generate the ultrasound device interaction indicator. The (zero force state) thickness of the acoustically translucent standoff layer may be selected according to a type of ultrasound imaging application or a target imaging anatomy. A thicker acoustically translucent standoff layer increases the field of view of the ultrasound device, but also increases a difficulty of imaging through bone and other high-attenuation materials. A thin acoustically translucent standoff layer, e.g. 3 mm, may therefore be preferable for, e.g., transcranial and apical cardiac applications, while a thicker acoustically translucent standoff layer, e.g. 7
mm, may be used for vascular applications.

Other factors might also affect the selected thickness of the acoustically translucent standoff layer. For example, an ease of measuring deformation increases as the thickness increases (as the compression travel is longer), but thicker acoustically translucent standoff layers have increased fragility and attenuation.

The acoustically translucent standoff layer may exhibit hysteresis behavior. Strain hysteresis is a well-known phenomenon that affects the determination of force/pressure from axial strain. The hysteresis effect becomes greater with successive force applications.

In some examples, the one or more characteristics of the acoustically translucent standoff layer may include hysteresis behavior, allowing this behavior to be taken into account when generating the ultrasound device interaction indicator.

In some examples, the processing system 220 may be configured to monitor hysteresis in the acoustically translucent standoff layer. For instance, the processing system may process the one or more characteristics of the acoustically translucent standoff layer and the ultrasound signal 235 to determine a measure of strain hysteresis of the acoustically translucent standoff layer. The processing system may then generate an indication that the acoustically translucent standoff layer needs replacing in response to a determination that the measure of strain hysteresis exceeds a predetermined hysteresis threshold, and output the indication at the output interface 222 (e.g. to the user output interface 260). The predetermined hysteresis threshold may be defined according to a desired level of uncertainty for the ultrasound device interaction indicator.

In some examples, the acoustically translucent standoff layer may be selected to reduce the effect of hysteresis. For instance, a stiffness of the acoustically translucent standoff layer will affect the hysteresis behavior. The acoustically translucent standoff layer may, for example, be formed of a material having a higher stiffness than the material used in traditional gel pads (e.g. Parker Aquaflex^{®} gel pads) in order to reduce the hysteresis effect and improve a reliability of the ultrasound device interaction indicator. However, increasing the stiffness of the acoustically translucent standoff layer also increases a difficulty in determining a measure of deformation, so there is a trade-off between reduction in hysteresis and ability to determine the measure of deformation.

In other examples, the hysteresis problem may be circumvented by only using the acoustically translucent standoff layer once. In other words, the acoustically translucent standoff layer may be a disposable standoff layer that is preserved in airtight packaging. This has the further advantage that the acoustically translucent standoff layer may be provided as a sterile standoff layer.

The acoustically translucent standoff layer 240 is positioned between the ultrasound device and the subject's skin 215. In some examples, the acoustically translucent standoff layer may comprise coupling means for fastening the acoustically translucent standoff layer to the ultrasound device. This aids installation and positioning of the acoustically translucent standoff layer.

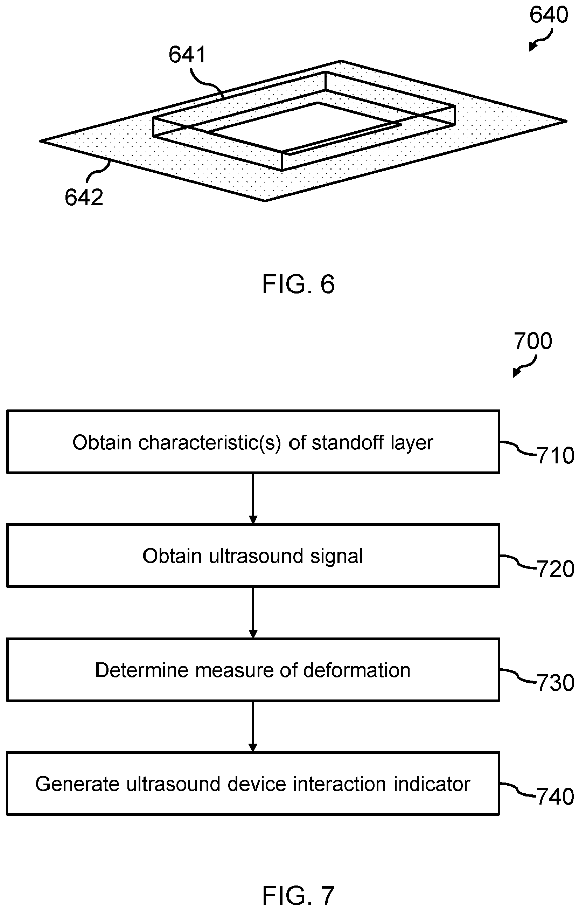

Fig. 6 illustrates an example acoustically translucent standoff layer 640 comprising coupling means. The example acoustically translucent standoff layer 640 comprises a gel pad 641 and an adhesive "skirt" 642 surrounding the perimeter of the acoustically translucent standoff layer. The adhesive skirt is attached to the gel pad, and has an adhesive surface that allows the acoustically translucent standoff layer to be mounted on an ultrasound device. The adhesive skirt has a cut-out at its center; the acoustically translucent standoff layer may be positioned on an ultrasound device such that the cut-out area corresponds to an ultrasound-emitting area of the ultrasound device, ensuring that the adhesive skirt does not affect the ultrasound signal acquired by the ultrasound device.

Other coupling means are envisaged, and suitable coupling means, such as clips, clamps and straps, will be apparent to the skilled person. For instance, the acoustically translucent standoff layer may be mounted in a rigid case that clips to the ultrasound probe.

Fig. 7 illustrates a computer-implemented method 700 for generating an ultrasound device interaction indicator, according to an embodiment of the invention. The ultrasound device interaction indicator is an indicator representative of a force or pressure exerted by an ultrasound device on a subject, and/or an orientation of the ultrasound device relative to the subject.

The method begins at step 710, at which one or more characteristics of an acoustically translucent standoff layer between a surface of the ultrasound device and the subject's skin are obtained.

At step 720, an ultrasound signal is obtained from the ultrasound device. The ultrasound signal is representative of a plurality of ultrasound echoes received by the ultrasound device from different depths for each of one or more scan lines.

At step 730, the ultrasound signal is processed to determine a measure of deformation of the acoustically translucent standoff layer.

At step 740, the measure of deformation and the one or more characteristics of the acoustically translucent standoff layer are processed to generate an ultrasound device interaction indicator.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (220) for generating an ultrasound device interaction indicator representative of a force or pressure exerted by an ultrasound device (230) on a subject (210) and/or an orientation of the ultrasound device relative to the subject, the processing system being configured to:
obtain, at an input interface (221), one or more characteristics (245) of an acoustically translucent standoff layer (240) between a surface of the ultrasound device and the subject's skin (215);
obtain, at the input interface, an ultrasound signal (235) from the ultrasound device, wherein the ultrasound signal is representative of a plurality of ultrasound echoes received by the ultrasound device from different depths for each of one or more scan lines;
process the ultrasound signal to determine a measure of deformation of the acoustically translucent standoff layer; and
process the measure of deformation and the one or more characteristics of the acoustically translucent standoff layer to generate the ultrasound device interaction indicator.

2. The processing system (220) of claim 1, wherein the one or more characteristics (245) of the acoustically translucent standoff layer (240) comprise at least one of: a thickness in a zero force state a thickness at a predefined non-zero compression force or pressure, and/or an elastic modulus.

3. The processing system (220) of claim 2, wherein the one or more characteristics (245) of the acoustically translucent standoff layer (240) further comprises a hysteresis behavior.

4. The processing system (220) of any of claims 1 to 3, wherein the processing system is configured to determine the measure of deformation by:
identifying a plurality of ultrasound echoes from each of the one or more scan lines in the ultrasound signal (235);
processing, for each scan line, the plurality of echoes from the scan line to determine a depth of a distal surface of the acoustically translucent standoff layer (240), wherein the distal surface of the acoustically translucent standoff layer is distal to the ultrasound device (230); and
processing the depth of the distal surface for each of the one or more scan lines to determine the measure of deformation.

5. The processing system (220) of claim 4, wherein the processing system is configured to determine the depth of the distal surface for each scan line by:
processing the plurality of echoes from the scan line to identify an echo for which a signal intensity value first exceeds an intensity threshold; and
selecting a depth of the identified echo as the depth of the distal surface.

6. The processing system (220) of any of claims 1 to 3, wherein the processing system is configured to determine the measure of deformation by:
processing the ultrasound signal (235) to generate an image comprising at least part of the acoustically translucent standoff layer (240); and
performing a cross-correlation of the generated image with a reference image to determine the measure of deformation.

7. The processing system (220) of any of claims 1 to 6, wherein the processing system is configured to generate the ultrasound device interaction indicator by selecting one of a plurality of predetermined ultrasound device interaction indicators.

8. The processing system (220) of any of claims 1 to 7, wherein the ultrasound device interaction indicator is a numerical value of the force or pressure exerted by the ultrasound device (230) on the subject (210).

9. The processing system (220) of any of claims 1 to 8, wherein the processing system is further configured to:
process the one or more characteristics (245) of the acoustically translucent standoff layer (240) and the ultrasound signal (235) to determine a measure of strain hysteresis of the acoustically translucent standoff layer; and, in response to a determination that the measure of strain hysteresis exceeds a predetermined hysteresis threshold:
generate an indication that the acoustically translucent standoff layer needs replacing; and
output, at an output interface (222), the indication that the acoustically translucent standoff layer needs replacing.

10. A system (200) for providing an ultrasound device interaction indicator representative of a force or pressure exerted by an ultrasound device (230) on a subject (210), the system comprising:
an ultrasound device configured to transmit and receive ultrasound, and to generate an ultrasound signal (235) representative of a plurality of received ultrasound from different depths for each of one or more scan lines;
an acoustically translucent standoff layer (240) positioned between a surface of the ultrasound device and the subject's skin (215); and
the processing system (220) of any of claims 1 to 9.

11. The system (200) of claim 9, wherein the acoustically translucent standoff layer (240) comprises an aqueous gel pad.

12. The system (200) of claim 10 or 11, wherein the acoustically translucent standoff layer (240) comprises coupling means (642) for fastening the acoustically translucent standoff layer to the ultrasound device (230).

13. The system (200) of any of claims 10 to 12, wherein a thickness of the acoustically translucent standoff layer (240) is selected according to a target imaging anatomy.

14. A computer-implemented method (700) for generating an ultrasound device interaction indicator representative of a force or pressure exerted by an ultrasound device (230) on a subject (210) and/or an orientation of the ultrasound device relative to the subject, the computer-implemented method comprising:
obtaining, at an input interface (221), one or more characteristics (245) of an acoustically translucent standoff layer (240) between a surface of the ultrasound device and the subject's skin (215);
obtaining, at the input interface, an ultrasound signal (235) from the ultrasound device, wherein the ultrasound signal is representative of a plurality of ultrasound echoes received by the ultrasound device from different depths for each of one or more scan lines;
processing the ultrasound signal to determine a measure of deformation of the acoustically translucent standoff layer; and
processing the measure of deformation and the one or more characteristics of the acoustically translucent standoff layer to generate the ultrasound device interaction indicator.

15. A computer program product comprising computer program code means which, when executed on a computer device having a processing system, cause the processing system to perform all of the steps of the method (700) according to claim 14.
